Europäisches Patentamt

European Patent Office

Office européen des brevets

(19)

(11)  **EP 1 365 345 A2**

(12)  **EUROPEAN PATENT APPLICATION**

(43) Date of publication:
26.11.2003 Bulletin 2003/48

(51) Int Cl.⁷: $G06F\ 19/00$

(21) Application number: 03252901.8

(22) Date of filing: 09.05.2003

(84) Designated Contracting States:
AT BE BG CH CY CZ DE DK EE ES FI FR GB GR
HU IE IT LI LU MC NL PT RO SE SI SK TR
Designated Extension States:
AL LT LV MK

(30) Priority: 22.05.2002 US 155616

(71) Applicant: Agilent Technologies, Inc.
Palo Alto, CA 94306 (US)

(72) Inventors:
• Kuchinsky, Allan
San Francisco, California 94127 (US)
• Moh, David
San Francisco, California 94131 (US)
• Adler, Annette Marie
Palo Alto, California 94301 (US)
• Thompson, Dean
Fort Collins, Colorado 80526 (US)
• Kincaid, Robert
Half Moon Bay, California 94019 (US)
• Vailaya, Aditya
Santa Clara, California 95054 (US)
• Hall, Deborah Elaine
San Francisco, California 94116 (US)
• Sampas, Nicholas M.
San Jose, California 95121 (US)

(74) Representative: Tollett, Ian et al
Williams Powell
Morley House
26-30 Holborn Viaduct
London EC1A 2BP (GB)

Remarks:
A request for correction of the description (the reference to the figures 1A to 1C being replaced by 1A to 1F) has been filed pursuant to Rule 88 EPC. A decision on the request will be taken during the proceedings before the Examining Division (Guidelines for Examination in the EPO, A-V, 3.).

(54)  **System and methods for visualizing diverse biological relationships**

(57)  An extensible, generalized architecture, an application programming interface, software and methods for building data overlays, as well as software, a system, methods and interface for building specific data overlays, with particular relevance to biological data.

FIG. 5

**Description**

The present invention pertains to a method and system for visualizing biological relationships and to software systems supporting the information synthesis activities of molecular biologists, in particular the activities of organizing, using, and sharing diverse biological information

The advent of new experimental technologies that support molecular biology research have resulted in an explosion of data and a rapidly increasing diversity of biological measurement data types. Examples of such biological measurement types include gene expression from DNA microarray or Taqman experiments, protein identification from mass spectrometry or gel electrophoresis, cell localization information from flow cytometry, phenotype information from clinical data or knockout experiments, genotype information from association studies and DNA microarray experiments, etc. This data is rapidly changing. New technologies frequently generate new types of data.

In addition to data from their own experiments, biologists also utilize a rich body of available information from internet-based sources, e.g. genomic and proteomic databases, and from the scientific literature. The structure and content of these sources is also rapidly evolving. The software tools used by molecular biologists need to gracefully accommodate new and rapidly changing data types.

Biologists use this experimental data and other sources of information to piece together interpretations and form hypotheses about biological processes. Such interpretations and hypotheses can be represented by narrative descriptions or visual abstractions such as pathway diagrams. To build interpretations and hypotheses, biologists need to view these diverse data from multiple perspectives. In particular, it is very important to validate the possible interpretations and hypotheses against the detailed, experimental results, in order to test whether the interpretations/hypotheses are supported by the actual data. An example of this would be to validate, test, or illustrate a putative pathway, represented in a pathway diagram, against gene expression data.

Although some tools have been developed for overlaying a specific type of data onto a viewer, they are very limited in their approach and do not facilitate the incorporation of disperse data types whatsoever. For example, a tool called EcoCyc [http://ecocyc.org]. is capable of overlaying gene expression data on pathways, but is limited to only gene expression data. Another example known as GeneSpring, by Silicon Genetics [http://www.sigenetics.com], is available for overlaying gene expression data on genomic maps, but again, is limited to this specific application.

Because of the vast scale and variety of sources and formats of these various types of data, an enormous number of variables must be compared and tested to formulate and validate hypotheses. Thus, there is a need for new and better tools that facilitate the comparisons of these data in formulating and validating/invalidating hypotheses.

An extensible architecture is provided that enables multiple data types to be overlaid upon multiple kinds of viewers, thus creating an understanding of biological context. This makes it much easier for product developers to incorporate new data types into the product, utilizing existing viewers. Also, it makes it easier for the user to identify correlations, disparities, and other patterns across multiple data types.

A method of visualizing biological relationships is disclosed to include providing first and second data sets of different type on a user interface; providing at least one of a pathway diagram, a textual story, or other map or diagram having elements corresponding to at least some of elements in the first and second data sets, on the user interface; correlating elements in the pathway diagram, textual story or other map or diagram with elements in the first and second data sets; mapping the correlated elements from the pathway diagram, textual story or other map or diagram to the corresponding elements in the first and second data sets; and selecting at least a portion of the data from the first data set and overlaying it on the pathway diagram, textual story or other map or diagram, wherein the values of the selected data are inserted into the pathway diagram, textual story or other map or diagram so that a user can compare the selected data values with corresponding hypothetical values existing in the diagram pathway, textual story or other map or diagram.

The data sets may be selected among various diverse types of data, including, but not limited to gene expression data, protein expression data, gene expression ratios, protein expression ratios, client patient information, Taqman data, cancer types data, breast cancer subtypes data, gene expression data from a microarray, protein abundance data generated by mass spectrometry and protein abundance data generated by gel electrophoresis.

Other maps or diagrams that may be employed include, but are not limited to gene expression profile plots, genome maps, chromosome maps, clustered heat maps, and tree views of hierarchical gene expression clusterings.

Further, a method of visualizing biological relationships is disclosed to include providing an experimental data set in a viewer on a user interface; providing at least one of a pathway diagram and a textual story on the user interface; correlating elements in the pathway diagram and/or textual story with elements in the experimental data set; mapping the correlated elements from the pathway diagram and/or textual story to the corresponding elements in the experimental data set; and selecting at least a portion of the experimental data and overlaying it on the pathway diagram or textual story, wherein the values of the experimental data are inserted into the pathway diagram and textual story so that a user can compare the experimental data values with corresponding hypothetical values existing in the diagram pathway and/or textual story.

The method may include iteratively stepping through the entire experimental data set by repeatedly selecting and overlaying successive portions of the experimental data set.

The method may further highlight elements of the pathway diagram and textual story elements to which the selected and overlayed data has been correlated.

The elements may be encoded so that different colors designate different activities, for example, proteins in the pathway diagram and textual story corresponding to up-regulated genes may be encoded as red, and proteins corresponding to down-regulated genes may be colored green.

The elements in the pathway diagram and textual story which do not correspond to the selected and overlayed data may be grayed out or colored gray.

The encoded values of the overlayed data may be optionally adjusted to accommodate for any and all of data distribution, switching between linear and logarithmic scales, filtering out certain subsets of the data, or changing the visual encoding used.

The adjustment of encoded values, when performed on a pathway, textual story or other map or diagram is automatically also performed on any remaining pathway, textual story, map or diagram.

A system for visualizing biological relationships from data selected among diverse data types is provided to include means for accessing data sets having diverse data types; means for selecting all or a portion of the data in each said data set; means for overlaying the selected data onto a pathway diagram, textual story or other map or diagram where the overlayed data can be compared; and means for visually displaying the overlayed data for visual comparison by a user.

Further, means for stepping through a series of portions of the data to display a series of overlayed data visualizations are provided.

A system for visualizing biological relationships from data selected among diverse data types is provided to include means for accessing data sets having diverse data types; and means for correlating the diverse data types across common attributes.

When the data sets include gene expression data sets and protein expression data sets, the means for correlating associates genes in the gene expression data sets with proteins that the respective genes encode, in the protein expression data sets, and means for encoding the correlation in a textual story, pathway diagram, or other map or diagram.

An interface for a user to manipulate encodings and transitions in overlayed biological data is provided to include an interactive display on a user interface; and means for modifying the functions or thresholds for color encoding of the overlayed data to accommodate different ranges and distributions in the experimental data.

The interface may further include any of means for converting the overlayed data between linear and logarithmic scales, means for filtering out subsets of the overlayed data, and/or means for changing the visual encoding used.

A computer readable medium is provided, carrying one or more sequences of instructions from a user of a computer system for visualizing biological relationships from data selected among diverse data types, wherein the execution of the one or more sequences of instructions by one or more processors cause the one or more processors to perform the steps of accessing data sets having diverse data types, and correlating the diverse data types across common attributes.

Further, the computer readable medium may be provided for carrying out the following further steps: providing the data sets in a viewer on a user interface; providing at least one of a pathway diagram, a textual story or other map or diagram on the user interface; correlating elements in the pathway diagram, textual story or other map or diagram with elements in the diverse data types; and mapping the correlated elements from the pathway diagram, textual story or other map or diagram to the corresponding elements in the data sets.

Still further, the computer readable medium may be provided for carrying out the steps of: selecting at least a portion of the data in one of the data sets and overlaying it on the pathway diagram, textual story, or other map or diagram, wherein the values of the selected data are inserted into and displayed on the pathway diagram, textual story or other map or diagram so that a user can compare the selected data values with corresponding hypothetical values existing in the diagram pathway, textual story or other map or diagram.

These and other objects, advantages, and features of the invention will become apparent to those persons skilled in the art upon reading the details of a number of preferred embodiments of the invention as more fully described below.

Fig. 1A shows an example of a main window of the present invention displayed on a graphical user interface (GUI) which includes gene array experimental results.

Fig. 1B shows an example of a main window of the present invention displayed on a graphical user interface (GUI) which includes protein abundance data obtained by mass spectrometry.

Fig. 1C shows an example of a main window of the present invention displayed on a graphical user interface (GUI) which includes gel data indicating protein abundance values.

Fig. 2 shows a user interface that includes interactive controls for performing modifications in the visual appearance of encoding of the data, according to the present invention.

Figs. 3A-3B illustrate one function of the interface shown in Fig. 2 for modifying the encoding.

Figs. 4A-4B illustrate another function of the interface shown in Fig. 2 for modifying the encoding.

Figs. 5-7 illustrate a flowchart representing a method of using the present invention.

Fig. 8 is a screen print showing an example of a Genome Map Viewer provided by NCBI.

Fig. 9 is a screen print showing an example of a Genome Map Viewer provided by UCSD.

Before the present system, tools and methods are described, it is to be understood that this invention is not limited to particular data sets, commands or steps described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within the invention. The upper and lower limits of these smaller ranges may independently be included or excluded in the range, and each range where either, neither or both limits are included in the smaller ranges is also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are now described. All publications mentioned herein are incorporated herein by reference to disclose and describe the methods, and/or algorithms in connection with which the publications are cited.

It must be noted that as used herein and in the appended claims, the singular forms "a", "and", and "the" include plural referents unless the context clearly dictates otherwise. Thus, for example, reference to "a step" includes a plurality or iterations of such steps and reference to "the pathway" includes reference to one or more pathways and equivalents thereof known to those skilled in the art, and so forth.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

Interpretations/hypotheses which are developed in either story form or diagrammatic form may be dependent upon many different cellular processes, genes, and various expressions of genes with resultant variations in protein abundance. Correlation and testing of data against these hypotheses is becoming increasingly more tedious and lengthy with the increased automation of the ways in which gene and other data is generated (e.g., microarrays, mass spectroscopy. etc.). The present invention provides a system, tools and methods for visualizing these correlations of data and hypotheses, through a mechanism called generalized data overlays.

It is also very useful to correlate experimental data with other representations of biological data, for example correlating gene expression data with genes on a chromosome map view, or with proteins in a pathway diagram. Still further, many biologists work with textual descriptions of hypotheses and interpretations of data; and would be useful to correlate experimental data with these textual representations, as well as graphical representations. In a data overlay according to the present invention, data from one view is encoded (e.g. color coded) and superimposed upon data items in a different view. Other examples of these kinds of overlays could include analytical plots, such as overlaying color coding or symbols onto log ratio plots. This would permit visualizing clinical data on textual representations, as well as graphical representations of the related subject matter.

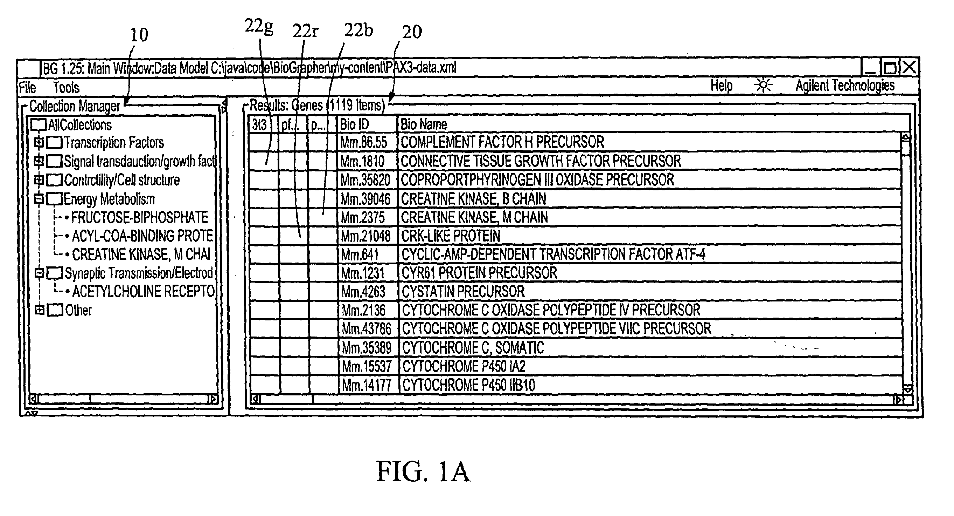

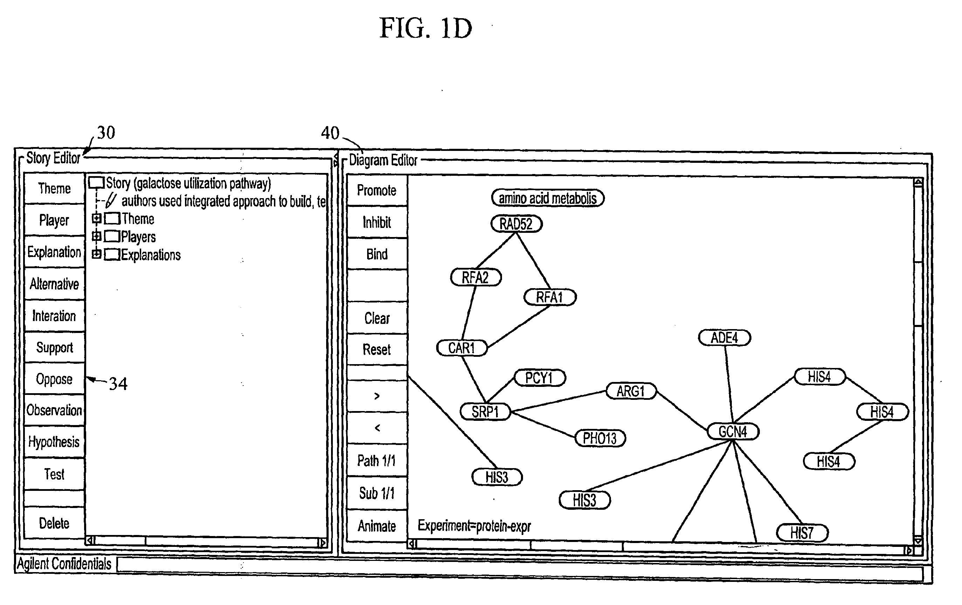

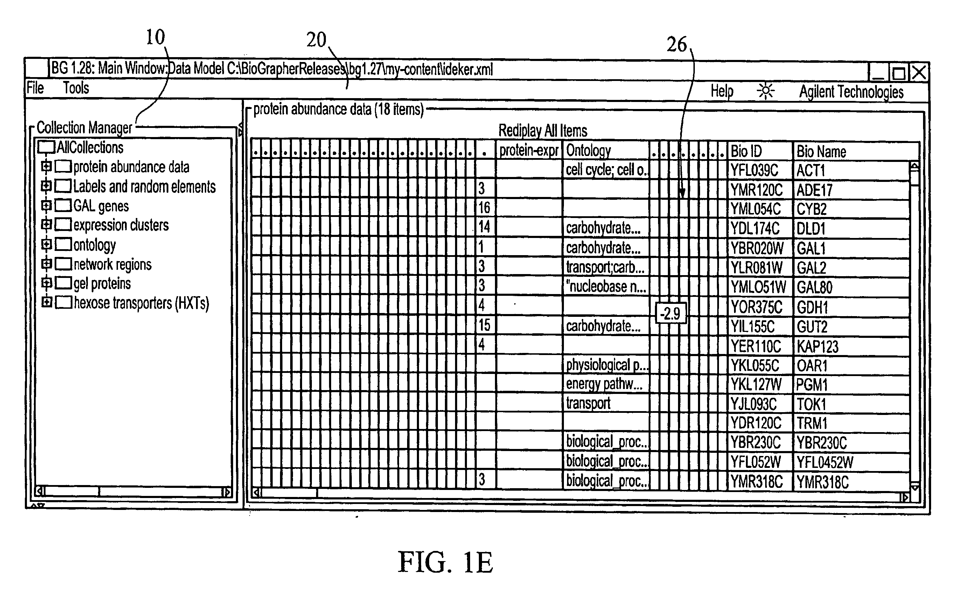

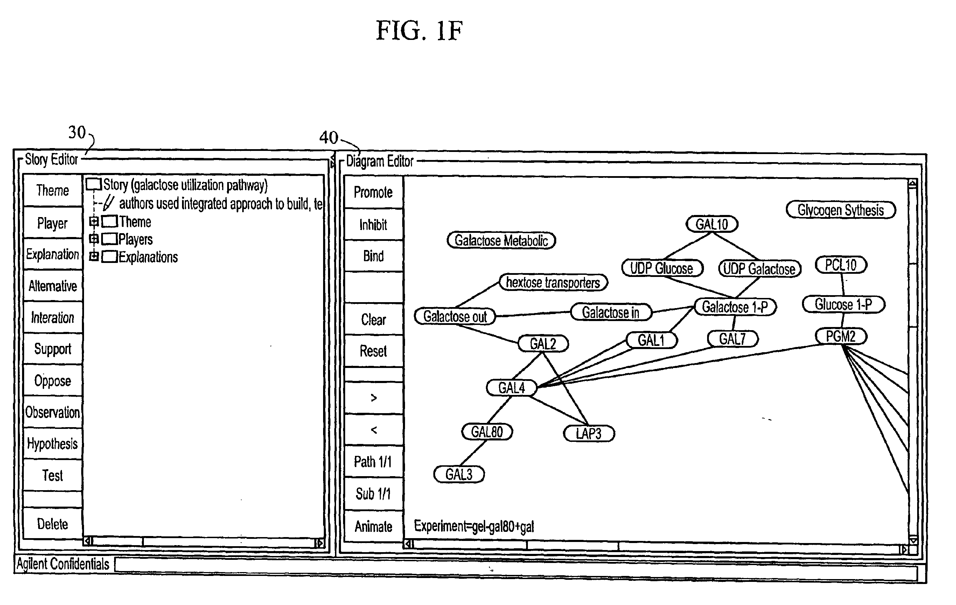

Fig. 1A shows an example of a main window of the present invention displayed on a graphical user interface (GUI). In this example, gene expression data, which is shown in the Results viewer pane 20 can be overlaid upon an interactive pathway diagram 42, such as that shown in the Diagram Editor 40, for example, and over a textual story representation, such as shown in the Story Editor 30, for example. The gene expression values are color-encoded, ranging from green (down-regulated gene, e.g., see 22g) to red (up-regulated gene, e.g., see 22r), with black being essentially neutral (i.e., neither up nor down-regulated, e.g., see 22b).

The present invention provides an extensible, generalized architecture and an application programming interface for building data overlays, as well as software for specific data overlays. The present invention facilitates and makes easier the incorporation of new and changing types of biological information and interpretations. For product developers, this means that it is easier to add new data types. When a new data type is needed by customers, for example protein abundance data from protein microarrays or mass spectrometry, the product developers can couple the new data with existing visualizations in a way that is much easier than with conventional, data type-specific viewers.

The present invention further facilitates the building of new and different visualizations (interpretations) for biological information, e.g., pathways, genome maps, biological ontologies, results from data mining or text mining, and the like.

From a user viewpoint, the present invention makes it easier to find patterns, correlations, and disparities across the different data types, for example, when gene expression data for a gene are inconsistent with the abundance data for the protein it encodes for. Additionally, communication and sharing of information between multiple users, possibly in different geographic, scientific, and/or methodological settings is enhanced. This is especially true for customers in pharmaceutical companies engaged in drug discovery.

The present invention provides generalized methods for visualizing these correlations of data and hypotheses, through mechanisms called generalized data overlays. In a data overlay, data from one view (for example, gene expression data or mass spectrometry data) is encoded (e.g. color coded) and superimposed upon data items in a different view (for example, a story or pathway diagram).

A story 32 as indicated in the story editor 30 utilizes narrative structure to represent the state of the biologist's hypotheses and understandings. Narrative structure provides a framework for organizing information about the interrelationships and biological interactions amongst items (e.g., genes, proteins, sequences, other gene products, etc.) and collections (e.g., groups of related items or information) in biological pathways. Biological stories can be thought of as templates for organizing and describing what is going on in the cell. A biological story can also be thought of as the representation of a hypothesis and the train of thought that produced that hypothesis. The user can piece together knowledge about a biological phenomenon and compose a biological story by using the story editor component 32 shown in Figs. 1A - 1C, which is described in more detail in commonly owned, co-pending U.S. Application Serial No. 09/863,115, filed May 22, 2001 and titled "Software System for Biological Storytelling", and commonly owned, co-pending application (Serial No, not yet assigned, Attorney's Docket No. 10020613-1), titled "Database Model, Tools and Methods for Organizing Information Across External Information Objects" and filed concurrently herewith, both of which are incorporated herein by reference thereto, in their entireties.

The narrative structure of the story is organized around a story grammar, drawn from cognitive psychology research. Briefly, a Story includes three main sections: a Theme, a list of one or more Players, and a set of Explanations. The Theme is a brief description of the overall gist of a biological story, such as might appear in the abstract of a journal article. The Players are the set of biological entities that play a role in the biological process being described in the Story, for example genes and proteins, or collections of genes and/or proteins. Explanations described the "plot" of the Story; they are essentially a set of evolving hypotheses about what processes may be occurring in a living cell, which are implied by the experimental data associated with the Story.

An Explanation can include one or more Interactions, basically steps in the process that is being described; for example, "PAX3-FKHR induces MY14". Different hypotheses can be represented by Alternatives, which specify different sets of possible Interactions. This is often useful in formative stages of an investigation, where there may be several plausible explanations for a particular biological phenomenon.

The biologist can document the reasoning behind Theme, Explanation, Interaction, and/or Alternative story elements via Support and Oppose story elements. For example, the biologist can use a Support element to provide a citation from the literature that provides supportive evidence for the claims made in the Alternative. Likewise, the biologist can use an Oppose story element to provide a citation from the literature that provides evidence that disputes a claim.

The Story Editor component 30 is a syntax-directed editor in which a biological story is represented by a tree structure. In this way, it is like an "outline processor". The tree appears on a canvas on the right side of the Story Editor component 32. Descriptions of biological phenomena are added to this tree, with nodes that correspond to the elements of narrative structure, i.e. Players, Explanations, etc. On the left side of the Story Editor component 30 is a set of buttons 34, which are used for adding nodes to (or deleting nodes from) the tree. Story nodes can be added to and deleted from the tree and textual descriptions can be added to story nodes in the tree. Textual descriptions can be to any node by either editing the node's label in place or by invoking an ObjectEditor interface that provides for detailed annotation of any node (again, see commonly owned, co-pending U.S. Application Serial No. 09/863115). Each story node represents an element of narrative structure: for example, a Player, Explanation or Interaction.

A story node can be added by pressing a button 34 in the Story Editor component 30, for example pressing the Player button to add a Player. For any story node in the story, there is a valid set of story nodes that can be nested below it. For example, it is valid to add a Player to the Players node, but not to the Theme node. When a story node is added, the buttons representing the valid story nodes that can be nested below it are enabled, whereas the non-valid story nodes are disabled (grayed out).

The user typically starts building up a biological story by specifying the Players in the story. Alternatively, an existing story may be imported into the present system and displayed in the Story Editor 30. The Players in a biological story can be either items or collections. Players may be added to a story by dragging and dropping (or cutting/copying and pasting) them from the Results viewer 20 and/or the Collection Manager 10, for example, when a story is being built or modified. Players can also be added by pressing the Player button 34 and then adding descriptive text to the node, as described above.

In its simplest form, the "plot" of a biological story represents a sequence or set of Explanations, which in turn contain a sequence or set of Interactions. The biologist creates Explanations by selecting the Explanation button in the Story Editor component 30, which causes an Explanation node to be added to the biological story. The investigator then enters a textual description of the biological Explanation by either editing the node's label in place or by invoking an ObjectEditor interface that provides for detailed annotation of any node.

The biologist creates Interactions by selecting the Interaction button in the Story Editor component 30, which causes an Interaction node to be added to the biological story. The investigator then enters a textual description of the biological Interaction by either editing the node's label in place or by invoking an ObjectEditor interface that provides for detailed annotation of any node.

In a situation where there may be more than one possible explanation for a sequence of events, alternative hypotheses for what is going on may be generated and tracked. This is often the case in the early phases of investigation, where there often are several possible explanations for a phenomenon. The user can add and keep track of all of the alternative hypotheses, and evolve them as the understanding of events becomes refined. To represent an alternative hypothesis, an Alternative node is added to the Explanations of the biological story, or to a specific Explanation or Interaction, by selecting the Alternative button 34. Then an alternative sequence of Explanations and/or Interactions can be added to that Alternative.

Since the user typically will have assumptions or evidence underlying different hypotheses, it is useful to keep track of these assumptions and evidence. The user can add a Support node to a Theme, Explanation, Player, Alternative, or Interaction by selecting the Support button (shown in Figs. 1A-1C), and inputting that information under the appropriate node. Similarly, information that contradicts a hypothesis may be tracked. This is done by adding an Oppose node in the same manner as described above with regard to a Support node, except that the Oppose button is selected to accomplish this task. Textual information may be added to the Support and/or Oppose node by either editing the node's label in place or by invoking an ObjectEditor interface that provides for detailed annotation of any node. Database and literature citations may be added to the Support and/or Oppose nodes by dragging and dropping a URL from a Web page onto a Support or Oppose node, or onto the ObjectEditor interface for that node.

Using the Story Editor component 30, the user can build up a structured textual representation of a biological story. Additionally, a biological pathway 42 may be constructed using the Diagram Editor 40. A biological pathway is a common way of representing a biological story pictorially. The present invention uses the Diagram Editor 40 to build or import a graphical analog of a biological story. An analogy can be drawn here to Computer-Aided Circuit Design (CAD) software, particularly to CAD schematic capture tools, in that the Diagram Editor 40 is used to sketch out a representation of the "circuitry" of a biological pathway.

The Diagram Editor 40 includes a canvas on the right and a set of buttons on the left for adding elements. Biological entities (e.g., items, collections, etc.) and their relationships (e.g., events) can be thought of as the "nouns" and "verbs" of the biological story. The pathway 42 can be built up by dragging/dropping items and/or collections onto the Diagram Editor panel when building a biological pathway 42. A graphical icon (e.g., see 44a, 44b, and 44c), representing the item or collection, appears at the drop point. There are a set of predefined "verbs" which are used to specify a relationship between "nouns", for example Inhibits, Promotes, or BindsTo.

Two "nouns" are connected with a "verb" by selecting the "verb" on the menu (e.g. by pressing a button 46 labeled Promote, Inhibit, or Bind), then drawing a line between the two graphical icons representing the "nouns." Drawing is accomplished by selecting the source node by clicking on it, pressing down on the mouse button for a "verb", dragging the mouse sprite over to the second item, then releasing the mouse button. A color-encoded arrow appears, connecting the two graphic icons, for example a red line represents the Inhibits "verb." "Verbs" in the Diagram Editor 40 are directional; that is, a red arrow running from item 44a to item 44b indicates that "a Inhibits b," but not the converse. Further detailed information regarding extracting semantic information from static diagrams to construct biological models which are editable by the Diagram Editor 40 and together with stories, data and other text, can be found in commonly owned, co-pending Application No. (application number not yet assigned, Atty Docket No. 10020150-1), titled "System and Methods for Extracting Semantics from Images", filed concurrently herewith, and incorporated herein, by reference thereto, in its entirety.

The present invention links the components of the Story Editor 30 and the Diagram Editor 40, so that any time an update is made to the Story in the Story Editor, the same change will be simultaneously made to any biological pathway in the Diagram Editor 40 in which that component is represented. That is, changes made to the underlying biological object referenced by a story node will be reflected in any diagram editor element that references that underlying biological object.

The present invention provides the ability to overlay items, collections, and biological stories with detailed experimental data, for comparison and validation or disproving of a hypothesis. Not only can the present invention overlay a set of expression levels on the Players in a biological story and highlight those genes whose expression levels exceed a certain threshold, but the same can be done on a biological pathway. More importantly, the present invention generalizes the applicability of the overlay feature, allowing other types of data to be overlayed, such as mass spectrometry data (representing protein abundance, for example), gel data, and Taqman data, to name a few varieties. This is analogous to the facilities in CAD tools for simulating circuit behavior; thus, the software provides a method for informally testing the hypotheses represented in biological stories and/or biological pathways against a variety of different data types. Further information regarding the representation of data in a local format that is common to all of the modes (e.g., Story, textual documents, biological model/pathways and experimental data) so that data can be readily compared, interchanged and edited among the modes is described in commonly owned, co-pending Application claiming priority from US 10/154,524 (Atty Ref. N13211), titled "System and Methods for Extracting Pre-Existing Data from Multiple Formats", filed concurrently herewith, and incorporated herein, by reference thereto, in its entirety.

For example, in Fig. 1A, the items 22 in the Results viewer 20 represent values from thousands of probes in a heat map produced by a microarray where, for example, test samples may be compared with references samples (e.g., diseased tissue versus "normal" tissue) under various conditions. Dye is mixed with the samples and they are hybridized to produce color-encoded results. Probes that are colored red indicate an up-regulation of the gene, those that are colored green indicate a down-regulation of the gene, and a black color represents neutral, i.e., substantially no up or down regulation. Various shades and intensities of green and red result, which indicate the relative degree of up or down regulation of any particular probe. In the example, there were 6000 rows in the matrix, although only a few have been shown for simplicity. Each column represents a different microarray experiment. For example, row 313 may knock out "gene A" to see how it effects the rest of the genes in the experiment, while row pf.. knocks out "geneB" and leaves "gene A" in the experiment, and so forth. As can be imagined, there are extremely numerous combinations of such runs that can be made in an effort to determine the role that each particular gene plays in a pathway.

In use, any column can be selected to overlay the values of that column onto the pathway in the Diagram Editor 40 and/or Story Editor 30. In the example shown in Fig. 1A, the 313 column has been selected as noted by the icon next to the column label "313". When a column is selected, any genes represented in that column are matched up with their representations in the pathway diagram 42 and the Story. A visual representation of this overlay is displayed, wherein the overlayed data shows up in its representative color on each of the icons in the pathway diagram as well as in the Story. This holds true for each icon in the pathway diagram that references an item in the experimental data, as well as each Player node in the StoryEditor that references an item in the experimental data.

A range of colors is mapped to a range of values in the data. Items that are correlated will have similar color schemes whereas items that are disparate or outliers would have different color schemes. When a value is outside of a predefined "normal range", the system may optionally identify the discrepancy by flashing the particular icon for which the disparity exists and/or sounding an audible alarm and/or displaying a text message, any and all of which alert the user to the discrepancy. This then provides the user the opportunity to closely review the values of the items involved, and to make proper annotation of the discrepancy, such as by entering an "Oppose" node as described above, for example. The ability to make and store such annotations is very valuable particularly in sharing information with other users working on the same pathway.

The user can repeat this process, a column at a time from the array values 22, thereby stepping through all of the data resultant from the microarray experiments and analyzing each column in the same manner to verify correlating data and annotate discrepancies and outliers, by visualizing the expression levels, color-coded on top of the icons for those items in the Diagram Editor and/or Story Editer. Such "simulations" can be useful, for example, in inferring relationships between items, such as causal relationships inferred by stepping through time course data.

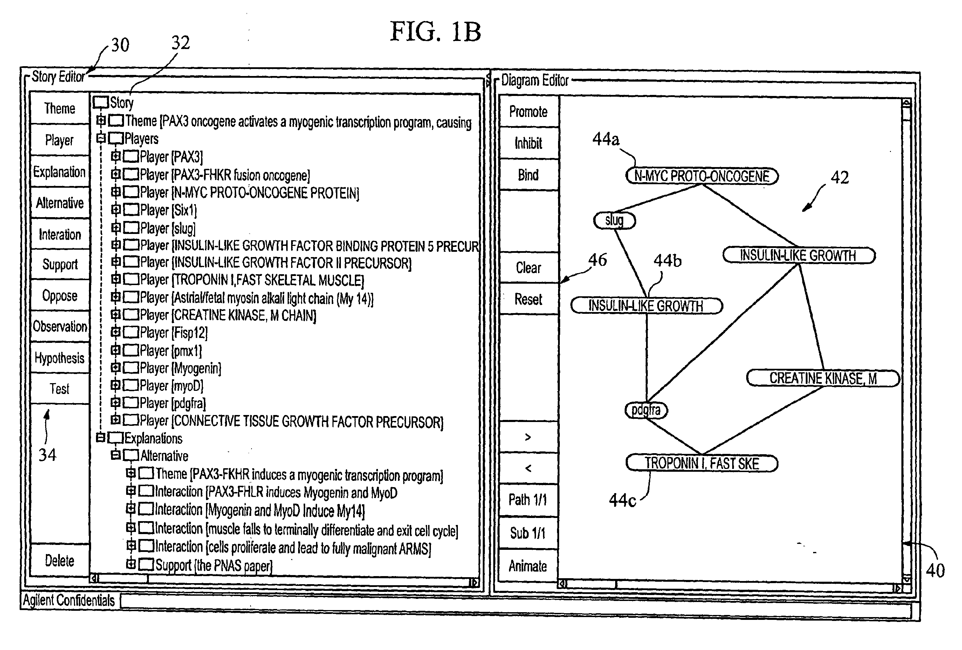

As noted, the present invention is capable of performing overlays of data from other diverse sources as well. For example, in Fig. 1B, mass spectrometry data is displayed in section 24 of the Results viewer which is indicative of relative protein abundance resultant from particular gene studies. This data 24 can also be overlayed on the pathway diagram 42 in Diagram Editor 40 or on the Players of the Story 32 in the Story Editor 30, at which time the overlayed data shows up in its representative color on each of the icons in the pathway diagram as well as in the Story which display the appropriate protein abundance values. Assuming that all of the overlayed values are within a predefined acceptable normal range about each of the values already contained in the Story and pathway diagram, the user can verify this visually by noting essentially similar color values. However, in addition to identifying correlations as just noted, this step is also useful in identifying disparities, outliers, and other surprises in the data. When a value is outside of a predefined "normal range", the system may identify the discrepancy by flashing and/or enlarging the particular icon for which the disparity exists and/or sounding an audible alarm and/or displaying a text message, any and all of which alert the user to the discrepancy. This then provides the user the opportunity to closely review the values of the items involved, and to make proper annotation of the discrepancy, such as by entering an "Oppose" node as described above, for example. The ability to make and store such annotations is very valuable particularly in sharing information with other users working on the same pathway.

Additionally, overlays on the Diagram Editor 40 can also serve to support or oppose interactions shown on the Diagram Editor 40. This can be especially useful for alerting a user to inconsistencies, such as where the expression levels of two nodes (e.g., two nouns) connected by an interaction (e.g., verb) do not coincide with the meaning of that interaction. For example, in the case where the Diagram Editor 40 contains nodes for "gene A" and "gene B" and contains an interaction indicating that "gene A promotes gene B", there would be an inconsistency if gene A's overlaid expression level was high and gene B's overlaid expression level was low. In such an instance, the present invention highlights the interaction, or otherwise alters it so as to indicate that there is an inconsistency. For example, the arrow from gene A to gene B (i.e., interaction) could be changed in style (e.g., from a solid line to a dashed line), changed in thickness, flashed, blinked or changed in color.

The user can repeat this process, a column at a time when multiple columns of protein abundance data are present, much in the same manner that the columns of array data were treated.

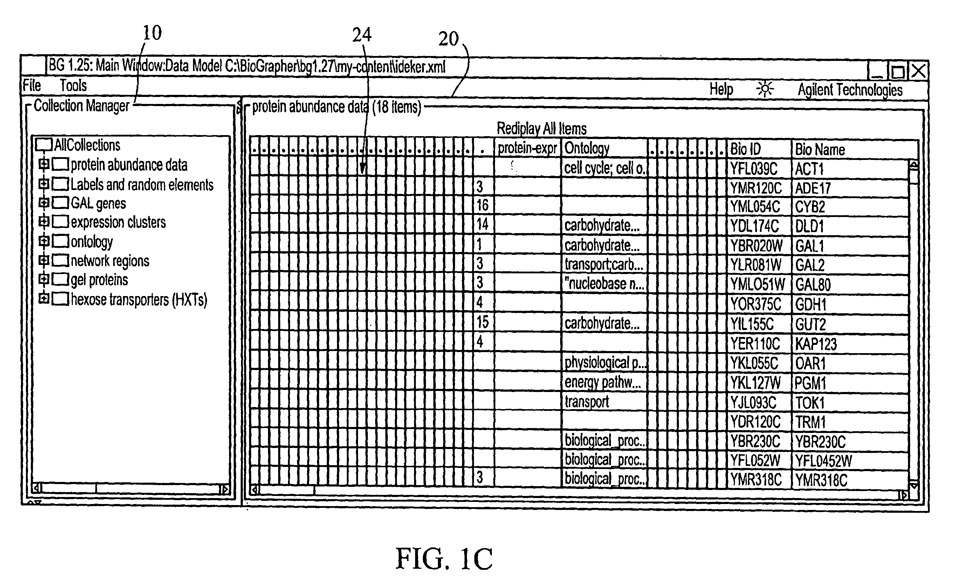

Still further, the present invention similarly allows overlay of gel data 26 representing protein abundance, as shown in Fig. 1C.

In addition to overlaying various data types over pathway diagrams and stories, as described above, the present invention may also use additional viewers to provide visual overlay data in additional formats. For example, a red or green band may be overlayed on a chromosome map indicating the relative position on the chromosome of the gene that the color represents.

The color encoding for gene expression data, relative protein abundance and other forms of data that a user may wish to overlay on a viewer as described herein although colored relative to each item in that particular experiment, are not normalized to one another. For this reason, one column of data may have items which are all very similar shades of red color for example, while others may have a readily recognizable distribution throughout the red green spectrum. Still further, some data sets may be valued in ratio values, where for example a color of a data point may be a value of the ratio of the test value to a control value, while others may be represented on a log scale, wherein the color is represented as a natural log function of the ratio. To account for these variations in data types, the present invention includes a user interface that allows the user to adjust the encodings of the data to change from the ratio scale to the log scale and vice versa, as well as to change the color spectrum of a column or an entire array, to give a more distinguishable color variation among the representative values.

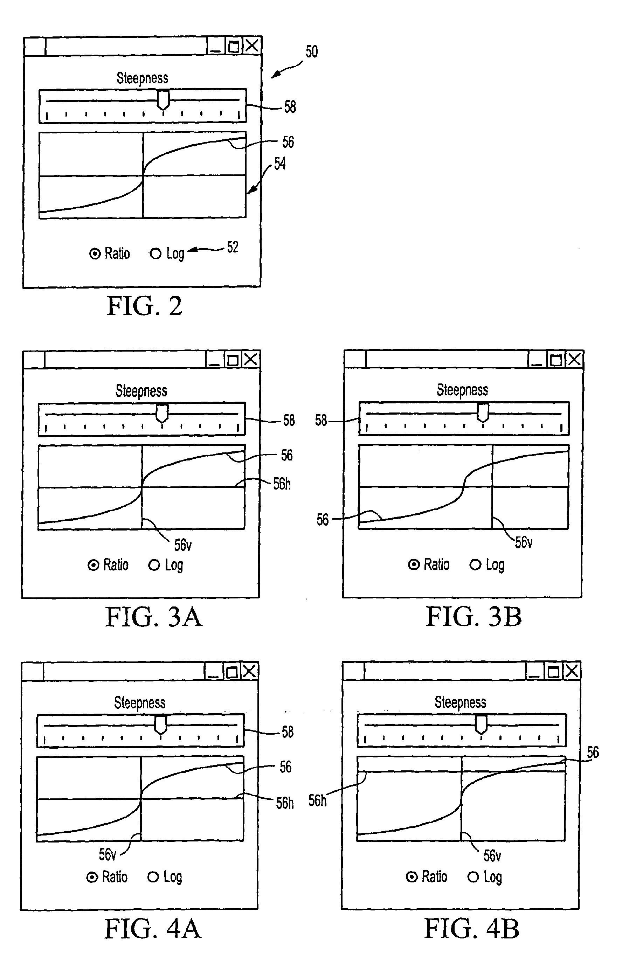

For example, Fig. 2 shows a user interface 50 that includes interactive controls for performing the modifications in the visual appearance of encoding of the data as described above. A virtual toggle switch 52 is provided to easily allow the user to convert the data back and forth between the ratio format and the natural log format by simply selecting above the desired format with the mouse or cursor and pressing enter. In the view shown, the ratio format has been selected. Above the virtual toggle switch 52 a data distribution adjuster 54 is provided. The data distribution adjuster provides a graphical representation of the distribution of the data selected, whether it be a column of an array, column of gel data, mass spectrometry data, or the like, or an entire array of data. The data curve 56 shown is representative of the data values of the population of the data selected. The data curve 56 illustrates the points along the function used to color encode the data values. The data curve is defined by the following equation:

$$y = 1/(1 + e^{-a(x-b)})$$

where

y = a value from 0 to 1, (the ordinate value);
a = a constant defining the steepness of the curve, which is determined by the steepness setting with slider 58 (described below);
x = the value of the data point selected, which, for example, may be a ratio of a fluorescence value characterizing the binding of a test sample to the fluoresced value characterizing the binding of a reference sample; and
b = a constant representing the midpoint of the curve (i.e., where x = 0).

Data points are mapped to color points along the curve. In the example shown, a well distributed population of data is represented, resulting in a full use of the range of the vertical color scale. The vertical color scale runs from +1 at the top of the scale to 0 at the bottom of the scale. The actual data values encoded by the color scale can be any positive or negative floating point number. The horizontal scale theoretically runs from -∞ to +∞ to include all of the data points selected. If the data curve were flatter, it could be advantageous to adjust the steepness of the curve to take full advantage of the color distribution. This can be accomplished, for example, with the slider 58 at the top of the screen, whereby sliding the selector switch to the left relatively flattens the S-curve, while sliding the selector to the right makes the curve steeper. Switching from the ratio scale to the log scale will redefine the curve to reflect the log values resultant from the converted ratio values.

Referring to Fig. 3A, a data curve 56 is shown which has a sufficiently steep curve to enable a reasonable distribution over the color spectrum, but a disproportionate number of the data values are in the red part of the spectrum. This is most easily ascertained by viewing the results viewer 20 which will show a predominant number of red data points with little to no green points displayed. In such a case, the user can drag and drop on the vertical axis 56v and move it to the right, as shown in Fig. 3B. By repositioning the vertical axis 56v, this also reconfigures the midpoint of range of data values defining the divider between green and red coloration, resulting in a relatively larger number of green data points, to aid in visually differentiating the data. By the same token, a shifting of the vertical axis to the left would produce more red data points, relatively speaking, in examples where this is helpful.

Fig. 4A shows an example where the midpoint of the curve is fairly well-placed but the distribution of green data points is fairly close to neutral (i.e. close to gray or black). In this case, the cursor or mouse can be used to drag and drop the horizontal axis 46h to place the left side of the curve more into the green spectrum for easier visual identification of the green data points, as shown in Fig. 4B. By the same token, a shifting of the horizontal axis 46h downwardly would place the red data points more into the red end of the spectrum.

Other changes to the data appearance that may be made are changing color-encoding to stipple patterns, selectively transforming size, shape, and/or rotation of nodes in the DiagramEditor, and the like. These functions are provided in the software architecture as a mechanism or mechanisms which are separate from a mechanism for t mapping the encodings to specific data values, e.g., continuous functions, intervals defined by thresholds, and categorical encodings. Any changes in the color encoding of any of the data are automatically made in all of the viewers to maintain an "apples to apples" comparison when the overlays are performed.

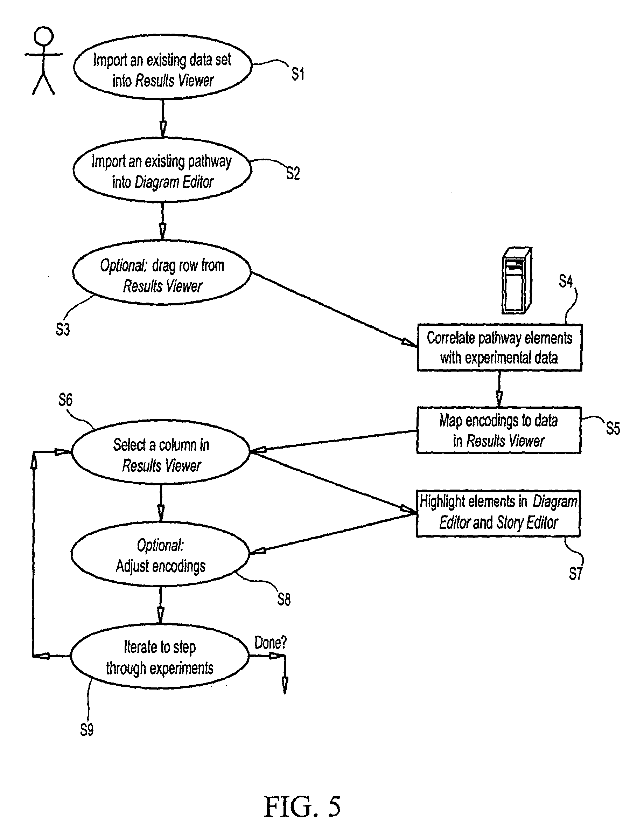

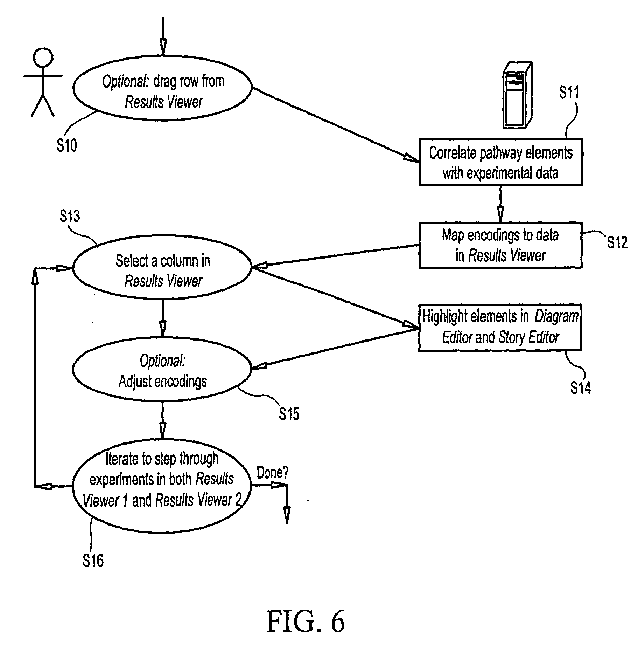

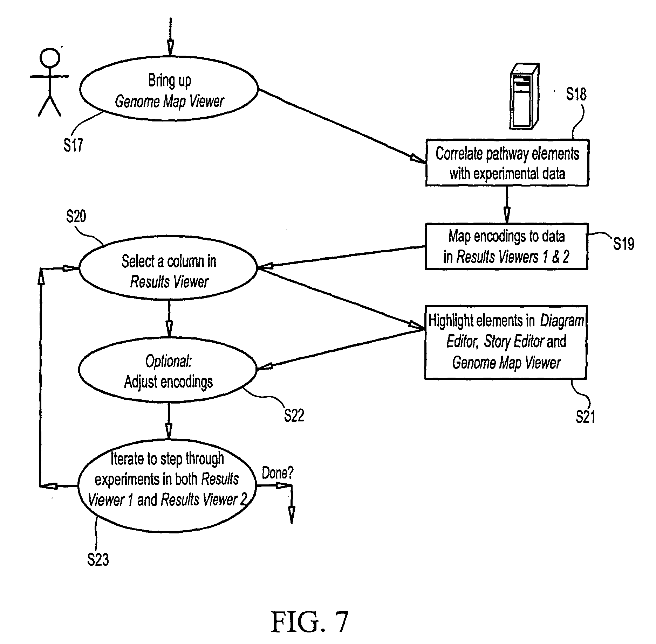

Referring now to Figs. 5-7, a flowchart representing a method of using the present invention is illustrated. In this example shown, the user first wants to import an existing pathway from a database and step through overlays of a number of gene expression data sets. While doing this, the user can also see the overlays of data upon a textual Story 32 that the user has developed which describes the user's current hypotheses about what is going on in the living cell. The user next wants to step through overlays of protein data related to the pathway (e.g. as derived from mass spectrometry). Finally, the user wants to look at these data from another perspective, for example overlaying them on a genome map. Specific examples that embody such a usage may include any or all of: gene expression and protein expression ratios simultaneously overlayed upon pathway diagrams; clinical patient information encoded on gene expression profile plots; TaqMan data overlayed upon a textual story hierarchy; cancer types overlayed upon a clustered heat map; and breast cancer subtypes overlayed upon a tree view of hierarchical gene expression clustering.

In step S1 of Fig. 5, the user imports an experimental data set into the Results viewer 20. The experimental data set can come from a database (or flat file) of gene expression data. In this example, the data represents a number of experiments on different samples using a particular DNA microarray. Each row represents a specific biological entity, in this case a gene (more specifically, the rows represent measurements of gene expression ratios for microarray probes that represent gene sequences). The columns represent the data from different experiments run for the genes.

In step S2, the user imports an existing pathway into the Diagram Editor 40. This pathway could come from an accessible pathway database, such as BIND DB, or might have been constructed by the user in the Diagram Editor, as mentioned above. The system utilizes an XML-based markup language, such as PathML, to layout the diagram from the imported pathway data.

Optionally, the user can extend the pathway diagram in step S3 by dragging a row in the Results viewer 20 into the Diagram Editor 40. The Diagram Editor 40 provides a library of connection primitives to enable the user to connect the new elements into the pathway diagram. These primitives represent relationships between the biological entities in the pathway diagram, such as "promotes", "inhibits", "binds" (which are also available via the buttons 46 for manual construction by the user, as described above), and are shown as lines and arrows.

Further optionally, the user can add a textual description of the hypotheses about what is going on in the cell. This could be done via the Story Editor component 30.

The system correlates the elements in the Diagram Editor 40 and the Story Editor 30 with specific rows in the gene expression data in step S4. The task here is to correlate genes in the Results Viewer 20 with the proteins they encode in the pathway. There are a variety of techniques that can utilize biological knowledge to make the correlations. The simplest case might be to use a common Accession Number in nucleotide and protein databases, or to follow links in LocusLink and/or GeneCards databases. Text mining software can be utilized, for example to infer correlations and relationships from articles in the research literature. A detailed description of tools and method for identifying such correlations and organizing them based upon a user's information is given in commonly owned, co-pending Application claiming priority from US 10/155,304 (Atty Ref. N13210), titled "Identification and Organization of New Information", filed concurrently herewith, and incorporated herein, by reference thereto, in its entirety. Still further, a simple name match between gene and protein could be utilized. An example of a correlation mechanism is described in more detail in co-pending, commonly owned Application (Application No. not yet assigned, Attorneys' Docket No. 10020142-1), filed concurrently herewith and titled "Biotechnology Information Naming System", which application is incorporated herein, in its entirety, by reference thereto. Additionally, commonly owned, co-pending Application No. 10/033,823, filed December 19, 2001 and titled "Domain Specific Knowledge-Based Metasearch System and Methods of Using", describes systems and methods of data mining, and is incorporated herein, in its entirety, by reference thereto.

The system next maps the encodings to the data in Results Viewer 20 in step S5. A simple case might involve mapping the gene expression data values to a color map running from green to red, as are seen in "heat map" diagrams of gene expression data. In constructing the encodings, the system takes into account the distribution of the data values, so that the range of data is mapped to fit with the range of encodings available, e.g., to accommodate for outliers and wide dynamic range. The system can then present to the user the results of this mapping in the Results Viewer 20.

At this time the user can begin stepping through the data using the data overlays according to the present invention. In step S6, the user selects a column in Results Viewer 20 to begin stepping through the data overlays. In response to the user's selection, in step S7 the system maps the column's values to elements in the pathway diagram 42 contained in the Diagram Editor 40 and in the Story Editor 30, and highlights the appropriate pathway and story elements accordingly. In this scenario, proteins corresponding to up-regulated genes are encoded as red, and proteins corresponding to down-regulated genes are colored green. Proteins corresponding to genes with normal regulation are colored black. Elements in the pathway diagram or story which do not correspond to specific rows in the Results Viewer 20 are colored gray.

Optionally, the User can adjust the encodings in step S8, via the user interface 50 described above, provided for this purpose. This could be used, for example, to accommodate for data distribution, to switch between linear and logarithmic scales, to filter out certain subsets of the data, or to change the visual encoding used, e.g. from color coding to stipple patterns.

The user iterates through steps S6-S8 (see step S9), as needed, to step through other experiments in the Results Viewer 20. When all of the data contained in the Results Viewer 20 has been stepped through, or, at the user's decision to do so, the user can import another experimental data set into a second Results Viewer 20, at step S10. In this example, the data is related to protein abundance data derived from mass spectrometry.

The system correlates elements and map encodings of the new data at steps S1 and S12, as in steps S4 and S5 above. Next, the user selects a column in the second Results Viewer 20 to begin stepping through the data overlays, see step S13. In step S14, the system maps the column's values to elements in the Diagram Editor 40 and Story Editor 30, and highlights the appropriate pathway and story elements accordingly, in the manner describe above with regard to step S7. The user is again given the option to adjust encodings in step S15, using the user interface 50. The user then iterates through steps S13-S15, as needed, to step through other experiments in the second Results Viewer 20. The user can also select a column or columns in the initial gene expression experiments Results Viewer 20, and the System will map values and highlight elements in the Story Editor 30 and Diagram Editor 40.

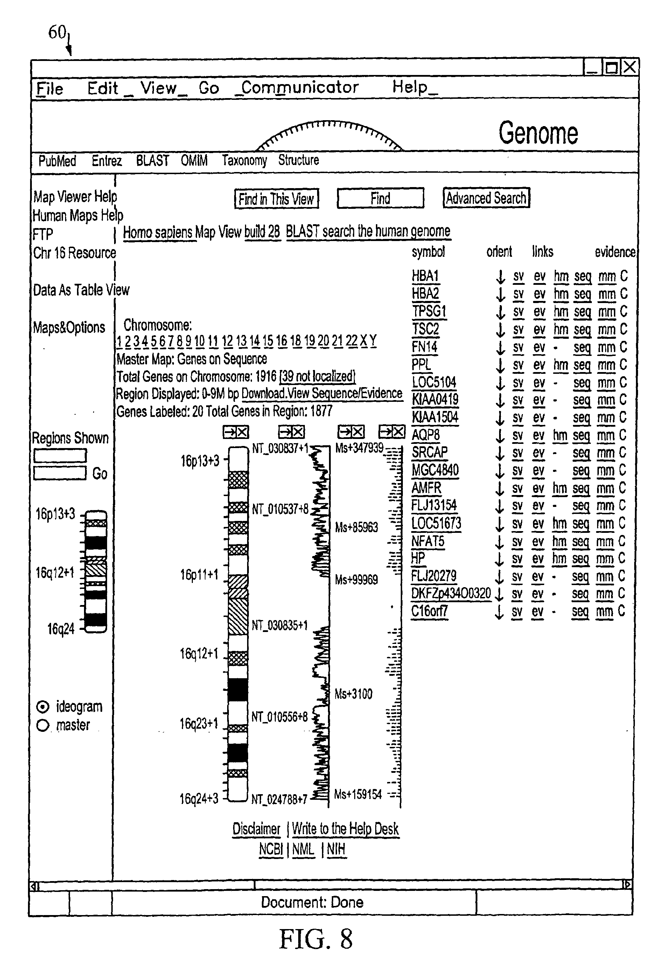

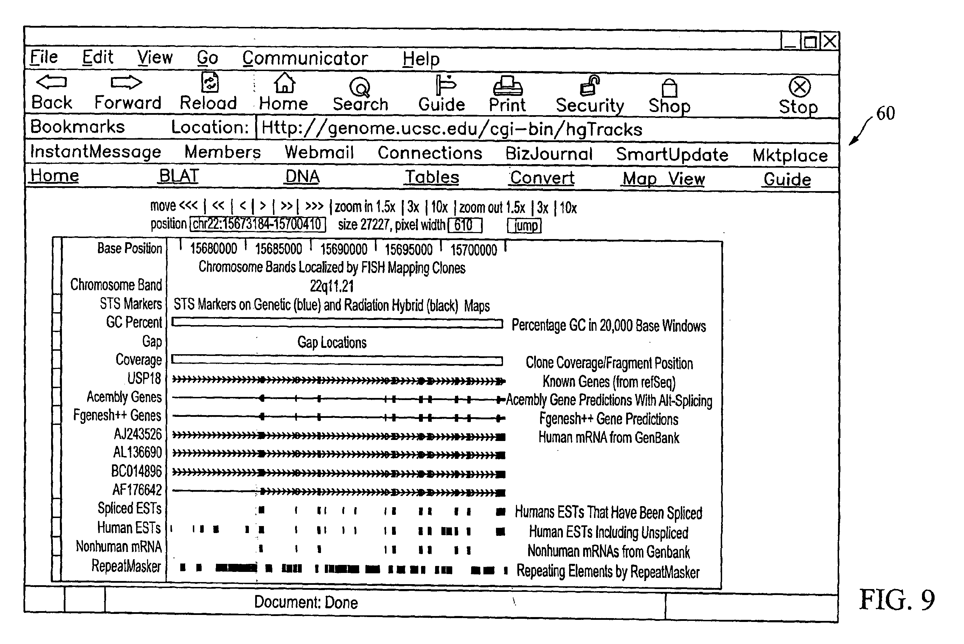

The user may bring up an additional viewer in step S17, in this example a Genome Map Viewer 60 (such as genome map viewers at http://ncbi.nlm.nih.gov or at http://genome.ucsd.edu, examples of which are shown in Figs.8 and 9, respectively, or the like). Other types of viewers, such as TreeMaps (a space-filling hierarchical data browser), parallel coordinates (a visualization for multidimensional information), etc. may be employed similarly. The system correlates the elements in the Genome Map Viewer with specific rows in the gene expression and protein abundance data in step S18, and maps encodings of the same in step S 19, in a manner as described with regard to steps S4 and S5 above.

Next, the user selects a column in the first or second Results Viewer at step S20 to begin stepping through the data overlays. The system maps the column's values to elements in the Diagram Editor, Story Editor and Genome Map Viewer, and highlights the appropriate pathway and story elements accordingly at Step S20, in the manner described above with regard to step S14. At step S22, the user has the option of adjusting encodings using the user interface 50, as described above. The user iterates through steps S2-S22, as needed, to step through other experiments in either of the two Results Viewers.

In this way, the user can identify disparities between the data sets and the graphical and textual interpretations. For example, a situation in which a gene is highly up-regulated and its corresponding protein is of low abundance might imply that there are some post-transcriptional modifications in play.

Note that while the usage described with regard to Figs. 5-7 has been as a linear sequence of steps, the usage in practice is much more exploratory. For example; the user might be jumping around from step to step, rather than following a linear sequence of steps. An important aspect of the present invention is that it is generalized, so that the user can step through multiple types of data and use multiple kinds of viewers. For example, the user could overlay the gene expression data on a genome map viewer. Or the user could map protein abundance or protein-protein interaction data upon a pathway viewer or genome viewer. Also, the user can identify disparities between data of different types, e.g. by highlighting proteins in the pathway diagram where the gene expression and protein abundance data are inconsistent. This can infer the existence of additional biological phenomena, such as post-transcriptional or post-translational modifications.

While the present invention has been described with reference to the specific embodiments thereof, it should be understood by those skilled in the art that various changes may be made and equivalents may be substituted without departing from the true spirit and scope of the invention. In addition, many modifications may be made to adapt a particular situation, data type, network, user need, process, process step or steps, to the objective, spirit and scope of the present invention. All such modifications are intended to be within the scope of the claims appended hereto.

**Claims**

1. A method of visualizing biological relationships, the method comprising the steps of:

   providing an experimental data set in a viewer (20) on a user interface (S1);
   providing at least one of a pathway diagram (S2) and a textual story on the user interface;
   correlating elements (S4, S11, S18) in the pathway diagram and/or textual story with elements in the experimental data set;
   mapping (S5, S12, S19) the correlated elements from the pathway diagram and/or textual story to the corresponding elements in the experimental data set;
   selecting at least a portion of the experimental data and overlaying it on the pathway diagram or textual story, wherein the values of the experimental data are inserted into the pathway diagram and textual story so that a user can compare the experimental data values with corresponding hypothetical values existing in the diagram pathway and/or textual story.

2. The method of claim 1, wherein the experimental data set comprises gene expression data from a microarray, protein abundance data generated by mass spectrometry, protein abundance data generated by gel electrophoresis, or Taqman data.

3. The method of claim 1 or claim 2, further comprising iteratively stepping (S9, S16, S23) through the entire experimental data set by repeatedly selecting and overlaying successive portions of the experimental data set.

4. The method of any of claims 1-3, wherein said selecting comprises selecting a column (S6, S13, S20) of data in the experimental data set.

5. The method of any of claims 1-4, further comprising highlighting elements (S7, S14, S21) of the pathway diagram and textual story elements to which the selected and overlayed data has been correlated, wherein proteins in the pathway diagram and textual story corresponding to up-regulated genes are encoded in a first color, and proteins corresponding to down-regulated genes are encoded in a second color that is visually distinct from said first color.

6. The method of any of claims 3-5, further comprising adjusting encoded values (S8, S15, S22) of the overlayed data, wherein said adjusting step comprises adjusting to accommodate for data distribution, adjusting to switch between linear and logarithmic scales, adjusting to filter out certain subsets of the data, or adjusting to change the visual encoding used.

7. The method of claim 6, wherein any adjustment of encoded values performed with regard to the pathway diagram is automatically also performed on the textual story and vice versa.

8. The method of any of claims 1-7, wherein the experimental data set comprises gene expression data, the method further comprising the steps of:

   providing an experimental protein abundance data set in a second viewer (20) on the user interface;
   correlating (S18) elements in the pathway diagram and/or textual story with elements in the experimental protein abundance data set, whereby elements in the gene expression data set are thereby also correlated with elements in the experimental protein abundance data set;
   mapping (S19) the correlated elements from the pathway diagram and/or textual story to the corresponding elements in the experimental protein abundance data set;
   selecting (S20) at least a portion of the experimental protein abundance data and overlaying it on the pathway diagram or textual story, wherein the values of the experimental protein abundance data are inserted into the pathway diagram and textual story so that a user can compare the experimental protein abundance data values with corresponding hypothetical values existing in the diagram pathway and/or textual story.

**9.** The method of claim 8, wherein at least a portion of the experimental gene expression data set and at least a portion of the experimental protein abundance data set are simultaneously overlaid upon the pathway diagram.

**10.** The method of claim 8 or claim 9, further comprising the steps of:

providing a third viewer (20) with a biological map upon which to overlay experimental data; correlating elements (S18) in the biological map with corresponding elements in the gene expression and protein abundance data sets; mapping (S19) the correlated elements from the biological map to the corresponding elements in the experimental protein abundance data set and experimental gene expression data set; selecting (S20) at least a portion of the experimental data in the gene expression data set or the protein abundance data set and overlaying it on the pathway diagram, textual story or biological map, wherein the overlay is also automatically performed on the pathway diagram, textual story and/or biological map at the same time; wherein the values of the selected experimental data are inserted into each of the pathway diagram, the textual story and the biological map, so that the user can compare the experimental protein abundance data values with corresponding hypothetical values existing in the diagram pathway, textual story and/or biological map.

**11.** A system for visualizing biological relationships from data selected among diverse data types, said system comprising:

means for accessing (20, 30, 40) data sets having diverse data types; means for selecting all or a portion of the data in each said data set; and means for overlaying the selected data onto a pathway diagram, textual story or other map or diagram where the overlayed data can be compared; and means for visually displaying the overlayed data for visual comparison by a user.

**12.** A computer readable medium carrying one or more sequences of instructions from a user of a computer system for visualizing biological relationships from data selected among diverse data types, wherein the execution of the one or more sequences of instructions by one or more processors cause the one or more processors to perform the steps of:

accessing data sets (S1, S2) having diverse data types; and correlating (S4) the diverse data types across common attributes.

**13.** The computer readable medium of claim 12, wherein the following further steps are performed:

providing the data sets in a viewer (20) on a user interface; providing at least one of a pathway diagram, a textual story or other map or diagram on the user interface (30, 40); correlating (S4) elements in the pathway diagram, textual story or other map or diagram with elements in the diverse data types; and mapping (S5) the correlated elements from the pathway diagram, textual story or other map or diagram to the corresponding elements in the data sets.

**14.** The computer readable medium of claim 13, wherein the following further steps are performed:

selecting at least a portion of the data in one of the data sets and overlaying it on the pathway diagram, textual story, or other map or diagram, wherein the values of the selected data are inserted into and displayed on the pathway diagram, textual story or other map or diagram so that a user can compare the selected data values with corresponding hypothetical values existing in the diagram pathway, textual story or other map or diagram.

BG 1.25: Main Window: Data Model C:\java\code\BioGrapher\my-content\PAX3-data.xml

File   Tools                    Help   ☼   Agilent Technologies

Collection Manager

☐ AllCollections
　⊞ ☐ Transcription Factors
　⊞ ☐ Signal transduction/growth fact
　⊞ ☐ Contrctility/Cell structure
　⊟ ☐ Energy Metabolism
　　　・・FRUCTOSE-BIPHOSPHATE
　　　─・ACYL-COA-BINDING PROTE
　　　・・CREATINE KINASE, M CHAI
　⊞ ☐ Synaptic Transmission/Electrod
　　　─・・ACETYLCHOLINE RECEPTO
　⊞ ☐ Other

Results: Genes (1119 Items)

| 3!3 | pf. | p... | Bio ID | Bio Name |
|-----|-----|------|--------|----------|
| | | | Mm.86.55 | COMPLEMENT FACTOR H PRECURSOR |
| | | | Mm.1810 | CONNECTIVE TISSUE GROWTH FACTOR PRECURSOR |
| | | | Mm.35820 | COPROPORTPHYRINOGEN III OXIDASE PRECURSOR |
| | | | Mm.39046 | CREATINE KINASE, B CHAIN |
| | | | Mm.2375 | CREATINE KINASE, M CHAIN |
| | | | Mm.21048 | CRK-LIKE PROTEIN |
| | | | Mm.641 | CYCLIC-AMP-DEPENDENT TRANSCRIPTION FACTOR ATF-4 |
| | | | Mm.1231 | CYR61 PROTEIN PRECURSOR |
| | | | Mm.4263 | CYSTATIN PRECURSOR |
| | | | Mm.2136 | CYTOCHROME C OXIDASE POLYPEPTIDE IV PRECURSOR |
| | | | Mm.43786 | CYTOCHROME C OXIDASE POLYPEPTIDE VIIC PRECURSOR |
| | | | Mm.35389 | CYTOCHROME C, SOMATIC |
| | | | Mm.15537 | CYTOCHROME P450 IA2 |
| | | | Mm.14177 | CYTOCHROME P450 IIB10 |

10   22g   22r   22b   20

FIG. 1A

## FIG. 1B

**Story Editor** (30, 32)

Buttons: Theme | Player | Explanation | Alternative | Interation | Support | Oppose | Observation | Hypothesis | Test (34) | Delete

- □ Story
  - ⊞□ Theme [PAX3 oncogene activates a myogenic transcription program, causing
  - ⊞□ Players
    - ⊞□ Player [PAX3]
    - ⊞□ Player [PAX3-FHKR fusion oncogene]
    - ⊞□ Player [N-MYC PROTO-ONCOGENE PROTEIN]
    - ⊞□ Player [Six1]
    - ⊞□ Player [slug]
    - ⊞□ Player [INSULIN-LIKE GROWTH FACTOR BINDING PROTEIN 5 PRECUR
    - ⊞□ Player [INSULIN-LIKE GROWTH FACTOR II PRECURSOR]
    - ⊞□ Player [TROPONIN I, FAST SKELETAL MUSCLE]
    - ⊞□ Player [Astrial/fetal myosin alkali light chain (My 14)]
    - ⊞□ Player [CREATINE KINASE, M CHAIN]
    - ⊞□ Player [Fisp12]
    - ⊞□ Player [pmx1]
    - ⊞□ Player [Myogenin]
    - ⊞□ Player [myoD]
    - ⊞□ Player [pdgfra]
    - ⊞□ Player [CONNECTIVE TISSUE GROWTH FACTOR PRECURSOR]
  - ⊟□ Explanations
    - ⊟□ Alternative
      - ⊞□ Theme [PAX3-FKHR induces a myogenic transcription program]
      - ⊞□ Interaction [PAX3-FHLR induces Myogenin and MyoD
      - ⊞□ Interaction [Myogenin and MyoD Induce My14]
      - ⊞□ Interaction [muscle falls to terminally differentiate and exit cell cycle]
      - ⊞□ Interaction [cells proliferate and lead to fully malignant ARMS]
      - ⊞□ Support [the PNAS paper]

**Diagram Editor** (40, 42)

Buttons: Promote | Inhibit | Bind | Clear (46) | Reset | > | < | Path 1/1 | Sub 1/1 | Animate

Nodes: N-MYC PROTO-ONCOGENE (44a) | slug | INSULIN-LIKE GROWTH | INSULIN-LIKE GROWTH (44b) | CREATINE KINASE, M | pdgfra | TROPONIN I, FAST SKE (44c)

EP 1 365 345 A2

BG 1.25: Main Window:Data Model C:\BioGrapher\bg1.27\my-content\ideker.xml

File    Tools                                                                    Help    ☼    Agilent Technologies

Collection Manager

☐ AllCollections
⊞☐ protein abundance data
⊞☐ Labels and random elements
⊞☐ GAL genes
⊞☐ expression clusters
⊞☐ ontology
⊞☐ network regions
⊞☐ gel proteins
⊞☐ hexose transporters (HXTs)

protein abundance data (18 items)

Rediplay All Items

| | protein-expr | Ontology | | Bio ID | Bio Name |
|---|---|---|---|---|---|
| | ( | cell cycle; cell o. | | YFL039C | ACT1 |
| 3 | | | | YMR120C | ADE17 |
| 16 | | | | YML054C | CYB2 |
| 14 | | carbohydrate... | | YDL174C | DLD1 |
| 1 | | carbohydrate... | | YBR020W | GAL1 |
| 3 | | transport;carb... | | YLR081W | GAL2 |
| 3 | | "nucleobase n... | | YMLO51W | GAL80 |
| 4 | | | | YOR375C | GDH1 |
| 15 | | carbohydrate... | | YIL155C | GUT2 |
| 4 | | | | YER110C | KAP123 |
| | | physiological p... | | YKL055C | OAR1 |
| | | energy pathw... | | YKL127W | PGM1 |
| | | transport | | YJL093C | TOK1 |
| | | | | YDR120C | TRM1 |
| | | biological_proc... | | YBR230C | YBR230C |
| | | biological_proc... | | YFL052W | YFL0452W |
| 3 | | biological_proc... | | YMR318C | YMR318C |

FIG. 1C

# FIG. 1D

EP 1 365 345 A2

Story Editor — 30

Theme

Player

Explanation

Alternative

Interation

Support

Oppose — 34

Observation

Hypothesis

Test

Delete

☐ Story (galactose utilization pathway)
┈ ∥ authors used integrated approach to build, te
⊞☐ Theme
⊞☐ Players
⊞☐ Explanations

Diagram Editor — 40

Promote

Inhibit

Bind

Clear

Reset

\>

\<

Path 1/1

Sub 1/1

Animate

amino acid metabolis

RAD52

RFA2

RFA1

CAR1

ADE4

PCY1

ARG1

SRP1

HIS4

HIS4

PHO13

GCN4

HIS3

HIS4

HIS3

HIS7

Experiment=protein-expr

Agilent Confidentials

BG 1.28: Main Window:Data Model C:\BioGrapherReleases\bg1.27\my-content\lideker.xml

File    Tools                                                                      Help    ☼    Agilent Technologies

Collection Manager

☐ AllCollections
⊞ ☐ protein abundance data
⊞ ☐ Labels and random elements
⊞ ☐ GAL genes
⊞ ☐ expression clusters
⊞ ☐ ontology
⊞ ☐ network regions
⊞ ☐ gel proteins
⊞ ☐ hexose transporters (HXTs)

protein abundance data (18 items)

Rediplay All Items

| protein-expr | Ontology | | Bio ID | Bio Name |
|---|---|---|---|---|
| | cell cycle; cell o. | | YFL039C | ACT1 |
| 3 | | | YMR120C | ADE17 |
| 16 | | | YML054C | CYB2 |
| 14 | carbohydrate... | | YDL174C | DLD1 |
| 1 | carbohydrate... | | YBR020W | GAL1 |
| 3 | transport;carb... | | YLR081W | GAL2 |
| 3 | "nucleobase n... | | YMLO51W | GAL80 |
| 4 | | -2.9 | YOR375C | GDH1 |
| 15 | carbohydrate... | | YIL155C | GUT2 |
| 4 | | | YER110C | KAP123 |
| | physiological p... | | YKL055C | OAR1 |
| | energy pathw... | | YKL127W | PGM1 |
| | transport | | YJL093C | TOK1 |
| | | | YDR120C | TRM1 |
| | biological_proc.. | | YBR230C | YBR230C |
| | biological_proc.. | | YFL052W | YFL0452W |
| 3 | biological_proc.. | | YMR318C | YMR318C |

10    20    26

FIG. 1E

FIG. 1F

FIG. 2

FIG. 3A

FIG. 3B

FIG. 4A

FIG. 4B

FIG. 5

FIG. 6

Bring up
*Genome Map Viewer*

S17

S18

Correlate pathway elements
with experimental data

Map encodings to data
in *Results Viewers 1 & 2*

S19

S20

Select a column in
*Results Viewer*

*Optional:*
Adjust encodings

S22

Highlight elements in *Diagram
Editor, Story Editor* and
*Genome Map Viewer*

S21

Iterate to step through
experiments in both *Results
Viewer 1* and *Results Viewer 2*

Done?

S23

FIG. 7

60

File    Edit_  View_  Go  _Communicator    Help_

Genome

PubMed    Entrez    BLAST    OMIM    Taxonomy    Structure

| | | |
|---|---|---|
| Map Viewer Help | [Find in This View]   [Find]   [Advanced Search] | |
| Human Maps Help | | |
| FTP | Homo sapiens Map View build 28  BLAST search the human genome | |
| Chr 16 Resource | | symbol  orient  links  evidence |

Data As Table View

Maps&Options

Chromosome:
1 2 3 4 5 6 7 8 9 10 11 12 13 14 15 16 18 19 20 21 22 X Y
Master Map: Genes on Sequence
Total Genes on Chromosome: 1916 [39 not localized]
Region Displayed: 0-9M bp Download.View Sequence/Evidence
Genes Labeled: 20 Total Genes in Region: 1877

| symbol | orient | links | | | evidence | |
|---|---|---|---|---|---|---|
| HBA1 | ↓ | sv ev | hm seq | mm | C |
| HBA2 | ↓ | sv ev | hm seq | mm | C |
| TPSG1 | ↓ | sv ev | hm seq | mm | C |
| TSC2 | ↓ | sv ev | hm seq | mm | C |
| FN14 | ↓ | sv ev | - seq | mm | C |
| PPL | ↓ | sv ev | hm seq | mm | C |
| LOC5104 | ↓ | sv ev | - seq | mm | C |
| KIAA0419 | ↓ | sv ev | - seq | mm | C |
| KIAA1504 | ↓ | sv ev | - seq | mm | C |
| AQP8 | ↓ | sv ev | hm seq | mm | C |
| SRCAP | ↓ | sv ev | - seq | mm | C |
| MGC4840 | ↓ | sv ev | - seq | mm | C |
| AMFR | ↓ | sv ev | hm seq | mm | C |
| FLJ13154 | ↓ | sv ev | - seq | mm | C |
| LOC51673 | ↓ | sv ev | hm seq | mm | C |
| NFAT5 | ↓ | sv ev | hm seq | mm | C |
| HP | ↓ | sv ev | hm seq | mm | C |
| FLJ20279 | ↓ | sv ev | - seq | mm | C |
| DKFZp434O0320 | ↓ | sv ev | - seq | mm | C |
| C16orf7 | ↓ | sv ev | - seq | mm | C |

Regions Shown

[      ]
[      ] Go

16p13+3

16q12+1

16q24

○ ideogram
○ master

16p13+3  NT_030837+1  Ms+347939

16p11+1  NT_010537+8  Ms+85963

NT_030835+1  Ms+99969

16q12+1

Ms+3100

16q23+1  NT_010556+8

16q24+3  NT_024788+7  Ms+159154

Disclaimer | Write to the Help Desk
NCBI | NML | NIH

Document: Done

FIG. 8

FIG. 9